(19) Europäisches Patentamt
European Patent Office
Office européen des brevets

(11) EP 0 771 589 B1

(12) EUROPÄISCHE PATENTSCHRIFT

(45) Veröffentlichungstag und Bekanntmachung des Hinweises auf die Patenterteilung:
**24.01.2001 Patentblatt 2001/04**

(51) Int Cl.⁷: **B01J 31/02**, C07C 37/20

(21) Anmeldenummer: **96115227.9**

(22) Anmeldetag: **23.09.1996**

(54) **Verfahren zur Herstellung von sulfonat- und mercaptogruppenhaltigen anorganischen Trägermaterialien zur Verwendung als Katalysatoren**

Process for the preparation of sulfonate and mercapto groups containing inorganic support material for use as catalysts

Procédé pour la preparation des matériaux de support minéral possédant des groupes sulfonates et mercaptan pour utilisation comme catalyseurs

(84) Benannte Vertragsstaaten:
**BE CH DE ES FR GB IT LI NL**

(30) Priorität: **31.10.1995 DE 19540497**

(43) Veröffentlichungstag der Anmeldung:
**07.05.1997 Patentblatt 1997/19**

(73) Patentinhaber: **Degussa-Hüls Aktiengesellschaft
60287 Frankfurt am Main (DE)**

(72) Erfinder:
- **Lansink-Rotgerink, Hans, Dr.
63864 Glattbach (DE)**
- **Wieland, Stefan, Dr.
63069 Offenbach (DE)**
- **Auer, Emmanuel, Dr.
60528 Frankfurt (DE)**

(56) Entgegenhaltungen:
**EP-A- 0 028 107**
**EP-A- 0 582 811**
**WO-A-92/16487**
**US-A- 3 172 916**
**EP-A- 0 319 327**
**EP-A- 0 693 470**
**GB-A- 1 506 226**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 771 589 B1

## Beschreibung

Die Erfindung betrifft ein Verfahren zur Herstellung von mit bifunktionellen schwefelhaltigen Organosiliciumverbindungen an der Oberfläche modifizierten oxidischen und silikatischen Feststoffen.

Derartige anorganische Feststoffe mit Organosiliciumverbindungen zu behandeln, ist aus dem Stand der Technik dem Grunde nach bekannt.

Dabei werden zum Beispiel Polysulfane (US-PS 4,514,231, DE-PS 33 14 742) oder Polyether-substituierte Siliciumverbindungen (US-PS 4,151,154) eingesetzt.

Die Problemstellung dieser Patente besteht jedoch immer darin festzustellen, auf welchem Weg man die Einarbeitbarkeit von anorganischen Füllstoffen in Kautschukmischungen verbessern könnte und welche gummitechnischen Eigenschaften sich gleichzeitig vorteilhaft ändern würden.

Die Aufgabe der vorliegenden Erfindung liegt darin, ein neues Verfahren zur Herstellung von Feststoffen zur verfügung zu stellen, die an der Oberfläche sowohl Sulfonat- bzw. Sulfonsäure als auch Mercaptogruppen aufweisen und als Katalysatoren zum Beispiel in Kondensationsreaktionen einsetzbar sind.

Aus dem Stand der Technik sind sulfonsaure organische Ionenaustauscher bekannt, die mit schwefelhaltigen, insbesondere Mercaptogruppen enthaltenden Verbindungen modifiziert werden, um die Aktivität und Selektivität als Kondensations-Katalysatoren zu steigern (EP-A - 0 630 878). Hier besteht jedoch die Gefahr, daß sich die schwefelhaltigen Verbindungen wieder von der Oberfläche ablösen und die Eigenschaften des entsprechenden Katalysators sich dann verschlechtern (EP-A - 0 583 712). Ein weiterer Nachteil dieses Katalysatortyps liegt darin, daß er vor dem Einsatz zum Beispiel in der Bisphenol-A-Synthese vorgequollen werden muß.

Aus der EP-A- 0 693 470 (Veröffentlichungsdatum 24.01.1996) sind Polyorganosiloxane bekannt, die Mercapto- und saure Gruppen enthalten. Man erhält diese Verbindungen durch Hydrolyse von Organosilanen, die aus Beispiel Alkoxygruppen und Mercaptogruppen enthalten, und die Sulfonierung von Mercaptogruppen.

Gegenstand der Erfindung ist ein Verfahren zur Herstellung von mit bifunktionellen Organosiliciumverbindungen an der Oberfläche modifizierten oxidischen und silikatischen Feststoffen wobei es sich um Organosiliciumverbindungen der allgemeinen Formel

$$[(RO)_y Si\text{-}R^1\text{-}SO_3^-]_x M^{x+} \qquad (I)$$

und der allgemeinen Formel

$$(RO)_y Si\text{-}R^2\text{-}SH \qquad (II)$$

handelt, in denen bedeuten:

$R^1$: eine lineare oder verzweigte Alkylengruppe mit 1 bis 12 C-Atomen, eine Cycloalkylgruppe mit 5 bis 8 C-Atomen

oder eine Einheit der allgemeinen Formeln

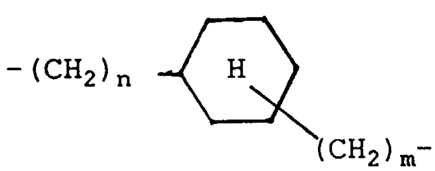

oder

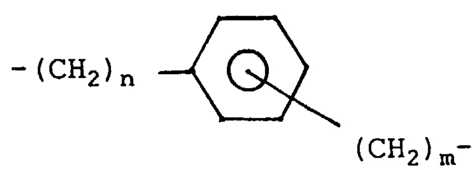

(III)

in der n bzw. m eine Zahl von 0 bis 6 ist und die Zahl der silicium- bzw. schwefelständigen Methylengruppen angibt, M gleich $H^+$ oder gegebenenfalls auch $NH_4^+$ oder ein Metallion mit einer Wertigkeit von x gleich 1 bis 4, wobei $H^+$ immer zu mindestens 5 % der Ionenaustauscherkapazität von (I) vorhanden ist,
y eine ganze Zahl zwischen 1 und 3, insbesondere 3,

$R^2$: gleich oder verschieden von $R^1$ sowie mit denselben Bedeutungen wie oben $R^1$

R: gleich oder verschieden Methyl, Ethyl oder Propyl, H.

Voraussetzung für die Herstellung der Feststoffe ist das Vorhandensein von OH-Gruppen an der Oberfläche. Diese treten mit den Alkoxygruppen der Verbindungen gemäß den Formeln (I) und (II) in Reaktion und bewirken somit die Bindung der katalytisch aktiven Gruppen $SO_3^- M^{x+}$ und SH an die Feststoffoberfläche, wobei $M^{x+}$ insbesondere $H^+$ bedeutet.

Aus diesem Grund wird die Gesamtzahl der umsetzbaren Alkoxygruppen der Verbindungen (I) und (II) die Zahl der an der Oberfläche der Feststoffe umsetzbaren OH-Gruppen in der Regel nicht überschreiten. Für bestimmte Einsatzzwecke bleibt jedoch ein gewisser Prozentsatz (1 - 30 %) der umsetzbaren OH-Gruppen bewußt erhalten. Der Begriff Oberfläche umfaßt dabei die gesamte, sich auch innerhalb zum Beispiel von Formkörpern der unterschiedlichsten Art befindlichen Flächen, insbesondere Oberflächen von Poren.

Im allgemeinen wählt man das molare Verhältnis der Verbindungen gemäß den Formeln (I) und (II) in der Weise, daß es, gemessen an den Gruppen $SO_3^-$ und SH, auf der Oberfläche zwischen 1 : 100 bis 100 : 1, insbesondere 1 : 10 bis 10 : 1 liegt, bevorzugt 1 : 3 bis 3 : 1. Das exakte Verhältnis wird insbesondere auch durch das Einsatzgebiet als Katalysator bestimmt.

Bevorzugt sind Verbindungen, in denen $R^1$ und $R^2$ dieselbe Bedeutung besitzen und für Propylen stehen und $M^{x+}$ und R in Formel (I) bevorzugt $H^+$ bzw. H bedeuten.

Die $SO_3$ -Gruppen werden in einer weiteren Ausführungsform zu 10 bis 90-Mol % durch Alkalimetallkationen neutralisiert.

Die geeigneten oxidischen und silikatischen Feststoffe können sowohl natürlicher als auch synthetischer Natur sein. Zu den natürlichen zählen insbesondere auch Clays. Bevorzugt sind synthetisch hergestellte Kieselsäuren, pyrogen oder gefällt oder insbesondere durch den Sol-Gel-Prozeß gewonnene, Aluminiumoxid, Titandioxid, Zirkondioxid sowie Mischoxide der genannten Verbindungen. Diese Feststoffe sind in feinteiliger Form zu modifizieren und dann zu den für das eingesetzte Verfahren vorteilhaften Formkörpern zu verarbeiten.

Besonders bevorzugt wird jedoch der Weg, bei dem man zum Beispiel durch Granulieren, Extrudieren oder Tablettieren gewonnene Formkörper mit den Verbindungen gemäß den Formeln (I) und (II) umsetzt.

In besonderen Fällen, wenn sich die Formkörper beispielsweise nicht als ausreichend abriebbeständig erweisen, erfolgt eine Behandlung dieser Materialien (Trägermaterialien) mit Verbindungen gemäß der allgemeinen Formel

$$Me(OR)_{2-4}R_{0-2} \text{ bzw. } Me(OR)_{2-3}R_{0-1} \qquad (IV),$$

in der bedeuten

Me: Si, Ti, Al
R: wie oben

bzw. deren Lösungen in Wasser, Ethylalkohol oder Methylalkohol, insbesondere Tetraorthosilikat oder dem technisch verfügbaren oligomeren Tetraethylsilikat 40 (= TES 40; bei vollständiger Hydrolyse bildet sich aus dem Oligomer $SiO_2$. Die gebildete $SiO_2$-Menge beträgt 40 Gew.-% der Einsatzmenge an TES 40). In gleicher Weise einsetzbar sind durch Alkylgruppen (1 bis 3 C-Atome) substituierte oder unsubstituierte Aluminate oder Titanate. Diese als nicht funktionelle Silane, Aluminate oder Titanate bezeichneten Verbindungen werden gegebenenfalls in einer Konzentration von 0 bis 50 Gew.-%, bezogen auf die Menge des Feststoffs, eingesetzt, bevorzugt von 1 bis 20 Gew.-%.

In einer besonders geeigneten Ausführungsform findet diese Behandlung vor der oder gleichzeitig mit der Modifizierung der Feststoffoberfläche mit wenigstens einer der Verbindungen gemäß den Formeln (I) und (II) statt.

Wird eine Behandlung der als Formkörper vorliegenden Feststoffe mit Verbindungen gemäß Formel (IV) als notwendig angesehen, legt man diese im allgemeinen in Form einer geeigneten Lösung vor, mischt den zu behandelnden Feststoff ein und erhitzt die Suspension unter Rühren auf Temperaturen zwischen 40 und 90°C, bevorzugt 60 bis 80°C. Nach 10 Minuten bis 4 Stunden trennt man den Feststoff vom Feinanteil (Abrieb bzw. aushydrolisiertes Oxid, zum Beispiel $SiO_2$ bei Verwendung von Tetraethylsilikat) ab und verarbeitet den so erhaltenen vorbehandelten Feststoff gegebenenfalls nach dem Trocknen bei 60 bis 140°C, bevorzugt 100 bis 130°C, weiter.

Die Konzentration von Verbindungen gemäß Formel IV in der Lösung bzw. Suspension beläuft sich im allgemeinen von 1 bis 40 Gew.-%, bezogen auf den eingesetzten Feststoff, und in Abhängigkeit von der Stabilität der verwendeten Formkörper.

Diese erste Verfahrensstufe kann man bei stabilen festen Formkörpern auslassen. Man kann sie in jedem Fall auch mit der ansonsten getrennt durchzuführenden zweiten Stufe kombinieren, indem man der alkoholischen Lösung von (IV) die Verbindungen gemäß Formel (I) und/oder Formel (II) zusetzt und die Behandlung des Feststoffes wie beschrieben durchführt.

Die Modifizierung der Oberfläche erfolgt jedoch in einer weiteren Variante nach dem ersten Verfahrensschritt a) in einer Weise, bei der man in einem zweiten Schritt gegegenenfalls wiederum eine Verbindung gemäß Formel (IV) und zusätzlich eine Verbindung gemäß Formel (I), vorzugsweise in Form einer wäßrigen oder alkoholischen Lösung, aufbringt.

Das molare Verhältnis der Sulfonat und Mercaptogruppen bewegt sich dann zwischen 100 : 1 und 1 : 100, bevorzugt 10 : 1 und 1 : 10, insbesondere 1 : 1. 3 und 3 : Die Modifizierung erfolgt durch Aufsprühen der Lösung(en) oder auch durch Tauchen der Formkörper in (eine) derartige Lösung(en). Die Temperatur liegt dabei zwischen 20°C und 140°C, insbesondere 20 und 100°C, wobei der Druck der Summe der sich bei der jeweiligen Temperatur einstellenden Summe der Partialdrucke oder dem Normaldruck entspricht. Gegebenenfalls schließt sich eine hydrothermale Behandlung, wie sie aus dem Stand der Technik bekannt ist, an.

Das so behandelte Trägermaterial (Feststoff) wird entweder unmittelbar für die weiteren Imprägnierungsschritte (Belegung) mit Organosiliciumverbindungen gemäß Formel (II), die in der Regel nicht in wäßrigem Medium löslich sind, eingesetzt oder aber vorher getrocknet, im allgemeinen bei Temperaturen von 70 bis 150°C.

Die Belegung des so erhaltenen Trägermaterials mit den Organosiliciumverbindungen gemäß Formel (II) erfolgt entweder durch Aufsprühen dieser Verbindungen, gegebenenfalls verdünnt mit einem organischen Lösungsmittel, insbesondere einem Alkohol mit 1 - 3 C-Atomen, oder durch Tauchen in einer entsprechenden Lösung dieser Verbindungen oder diesen selbst. Dabei ist in jedem Fall für eine ausreichende Durchmischung zu sorgen.

Das so erhaltene Material wird nach der Umsetzung bei 20 bis 60°C gegebenenfalls zum Beispiel mit den genannten Alkoholen gewaschen, um überschüssige nicht abreagierte Organosiliciumverbindungen zu entfernen und anschließend bei Temperaturen zwischen 70 und 150°C, gegebenenfalls bis 250°C getrocknet.

Wählt man den Weg, das Trägermaterial entweder mit den Reinsubstanzen oder mit deren Lösungen zu besprühen, ist es der Feinabstimmung durch den Fachmann überlassen, wie intensiv er zum Beispiel bei Vorliegen von Formkörpern in Anbetracht von deren Stabilität die Durchmischung gestaltet.

Technisch machbar sind beispielsweise auch Vorgehensweisen, bei denen in einer ersten Stufe die Durchmischung oder Homogenisierung erfolgt und dann die homogenisierte Mischung zum Beispiel in einem weiteren vorgeheizten Mischer der eigentlichen Umsetzung unterworfen wird.

Die Organosiliciumverbindungen gemäß den Formeln (I) und (II) sind in einer weiteren Variante auch gemeinsam auf das Trägermaterial aufzubringen. In diesem Fall verwendet man vorzugsweise eine alkoholische wäßrige Lösung, die gegebenenfalls einen bekannten Emulgator enthält.

Die Konzentration der beiden Verbindungen wird bei der Modifizierung in allen Fällen so eingestellt, daß sie auf dem zu modifizierenden Material, bevorzugt in einem molaren Verhältnis von 1 : 10 bis 10 : 1, insbesondere 1 : 3 bis 3 : 1, vorliegen.

Der Gesamtgehalt der Organosiliciumverbindungen gemäß den Formeln (I) und (II) beläuft sich dann im allgemeinen auf 0,1 bis 70 Gew.-%, bevorzugt 1 bis 10 bezogen auf den Feststoff.

Für die katalytischen Eigenschaften ist der Gesamtschwefelgehalt oder auch die durch Titration der Sulfonsäuregruppen bestimmte Säurekapazität und die sich als Differenz zum Gesamtschwefelgehalt ergebende Mercaptogruppenbelegung ausschlaggebend.

Die Feststoffe haben sich als gute Kondensationskatalysatoren für sauer katalysierte Reaktionen erwiesen. Insbesondere bei der Bisphenol-A-Synthese werden bei ihrem Einsatz hohe Umsätze und Selektivitäten erzielt.

## Beispiel 1

700 ml (350 mmol) einer 0,5 M wäßrigen Lösungen von $(HO)_3Si\text{-}CH_2CH_2CH_2SO_3H$(3-Trihydroxysilylpropylsulfonsäure) und 76 g Tetraethylorthosilikat 40 ("TES 40", vorkondensiertes Tetraethylorthosilikat, entsprechend der Bildung von 505 mmol $SiO_2$) werden in einem 3 l Doppelmantelglasgefäß vorgelegt und auf 80°C hochgeheizt. 200 g $SiO_2$-Träger (Grace-Typ C 15) werden chargiert und 3 Stunden unter vorsichtigem Rühren mit der Silanmischung umgesetzt. Danach werden die imprägnierten Feststoffkugeln von Feinanteil (Abrieb bzw. aushydrolisiertes $SiO_2$) abgetrennt, die imprägnierten $SiO_2$-Kugeln zweimal mit Wasser gewaschen und abfiltriert sowie anschließend 12 Stunden unter Stickstoffatmosphäre bei 135°C getrocknet. Es werden 223 g eines glänzenden kugelförmigen Feststoffes isoliert. Durch Titration des Feststoffs mit 1 n Natronlauge gegen Phenolphthalein wird eine Säurekapazität von 0,25 mmol/g Feststoff bestimmt. Elementaranalytisch wird ein Schwefelgehalt von 1,7 % bestimmt.

50 g des so vorbehandelten sulfonsäuregruppenhaltigen Trägers werden anschließend in Ethanol eingetragen und mit der vierfachen Menge, bezogen auf die vorhandenen Sulfonsäuregruppen, mit Mercaptopropyltrimethoxysilan (50 mmol entsprechend 9,8 g) versetzt und 3 Stunden am Rückfluß gerührt. Danach wird der Feststoff durch Filtration isoliert (kein Abrieb mehr festzustellen) und mit Ethanol gewaschen. Nach Trocknung des Materials unter Stickstoff bei 135°C über einen Zeitraum von 12 Stunden werden 55 g Produkt erhalten, welches einen Schwefelgehalt von 3,3 Gew.-% aufweist (Zunahme des Schwefelgehaltes um 1,6 % entsprechend 57 % der Theorie).

**Beispiel 2**

300 g kugelförmigen $\gamma$-Aluminiumoxids (Durchmesser 1,7 - 2,3 mm; BET-Oberfläche 185 $m^2/g$; Porenvolumen von 0,6 ml/g) werden in einem rotierenden Behälter (Mischer) vorgelegt und mit 180 ml (144 mmol) einer 0,8 molaren wäßrigen Lösung von $(HO)_3Si\text{-}CH_2CH_2CH_2SO_3H$ (3-Trihydroxysilylpropylsulfonsäure) besprüht. Der Sprühvorgang dauert 30 Minuten, danach werden die Pellets bei 120°C im Vakuum getrocknet. Die erhaltenen 325 g vorbehandelten Trägers werden daraufhin in einem rotierenden Behälter mit 100 ml einer ethanolischen Lösung von Mercaptopropyltriethoxysilan (28,6 g entsprechend 120 mmol gelöst in 71 ml Ethanol) besprüht und danach erneut bei 120°C im Vakuum getrocknet. Es werden 340 g eines glänzenden sehr abriebbeständigen kugelförmigen Materials erhalten.

| | |
|---|---|
| Schwefelgehalt | (Theorie in Klammern):2,3 % (2,5 %) |
| Säurekapazität | (Theorie in Klammern):0,4 mmol/g (0,42 mmol/g) |

**Beispiel 3**

200 g verformtes $TiO_2$ werden wie in Beispiel 1 beschrieben behandelt. An dem isolierten Feststoff werden folgende analytische Werte bestimmt:

| | |
|---|---|
| Schwefelgehalt: | 1,2 % |
| Säurekapazität | 0,01 mmol/g |

**Beispiel 4**

In einer Rührapparatur werden 10 g eines mit Sulfonsäure- und Mercaptogruppen imprägnierten und getrockneten $SiO_2$-Tägermaterials (Grace C15), das wie in Beipiel 1 beschrieben hergestellt wird, mit 70,58 g (0,75 mol) Phenol und 4,36 g (0,075 mol) Aceton bis zu einem Acetonumsatz von 99 % gerührt. Man erhält eine gaschromatographisch bestimmte Ausbeute an p/p-Bisphenol-A von 96,4 % bei einer Selektivität für p/p-Bisphenol-A von 97,2 % bezogen auf eingesetztes Phenol.

**Beispiel 5**

Ein zylindrischer Reaktor (Durchmesser 45 mm x Länge 170 mm) wird mit 100 g eines wie in Beispiel 1 beschriebenen imprägnierten $SiO_2$-Trägers vom Typ Grace C15 gefüllt und mit 50 g/h eines Phenol/Aceton-Gemisches im Molverhältnis 10 : 1 durchströmt. Bei einer WHSV von 0,3 $h^{-1}$ beträgt der Acetonumsatz 96,9 %, die Ausbeute an p/p-Bisphenol-A 93,8 % und die Selektivität für p/p-Bisphenol-A, bezogen auf Phenol, 95,5 %. Die Menge nicht isomerisierbarer Nebenprodukte ist geringer als 0,1 %.

**Patentansprüche**

1. Verfahren zur Herstellung von mit bifunktionellen Organosiliciumverbindungen an der Oberfläche modifizierten oxidischen und silikatischen Feststoffen, wobei es sich um Organosiliciumverbindungen der allgemeinen Formel

$$[(RO)_ySi\text{-}R^1\text{-}SO_3^-]_xM^{x+} \qquad (I)$$

und der allgemeinen Formel

$$(RO)_ySi\text{-}R^2\text{-}SH \qquad (II)$$

handelt, in denen bedeuten:

$R^1$: eine lineare oder verzweigte Alkylengruppe mit 1 bis 12 C-Atomen, eine Cycloalkylengruppen mit 5 bis 8 C-Atomen

oder eine Einheit der allgemeinen Formeln

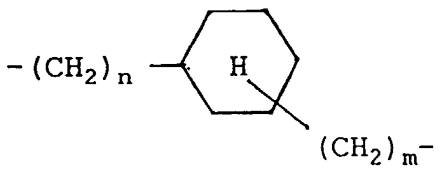 oder 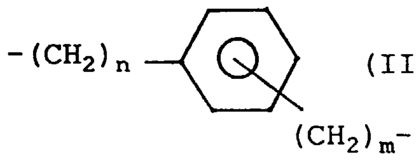 (III)

in der n bzw. m eine Zahl von 0 bis 6 ist und die Zahl der silicium- bzw. schwefelständigen Methylengruppen angibt,

$M^+$ gleich $H^+$ oder gegebenenfalls $NH_4^+$ oder ein Metallion mit einer Wertigkeit von x gleich 1 bis 4, wobei $H^+$ immer zu mindestens 5 % der Ionenaustauschkapazität von (I) vorhanden ist,
y eine ganze Zahl zwischen 1 und 3,

$R^2$: gleich oder verschieden von $R^1$, sowie mit denselben Bedeutungen wie oben $R^1$

R: gleich oder verschieden Methyl, Ethyl oder Propyl, H,

**dadurch gekennzeichnet,**
daß man

a) gegebenenfalls den als Formkörper vorliegenden Feststoff (Trägermaterial) durch Tauchen oder Besprühen mit einer Verbindung der allgemeinen Formel

$$Me(OR)_{2-4}R_{0-2} \text{ bzw. } Me\,(OR)_{2-3}R_{0-1} \qquad (IV),$$

Me: Si, Ti, Al
R: wie oben

imprägniert, diesen nach Abtrennen von der gegebenenfalls vorliegenden flüssigen Phase bei 60 bis 140°C trocknet und

b) das Trägermaterial bei einer Temperatur von 20 bis 140°C mit einer wäßrigen Lösung von Organosiliciumverbindungen gemäß der Formel (I) durch Tauchen in die Lösung oder Besprühen mit ihr bei Normaldruck oder einem Druck umsetzt, der der Summe der sich bei der jeweiligen Temperatur einstellenden Partialdrucke entspricht,
das imprägnierte Trägermaterial abtrennt und gegebenenfalls bei 60 bis 140°C trocknet, gegebenenfalls hydrothermal nachbehandelt und

c) das so erhaltene Trägermaterial mit einer Lösung einer Organosiliciumverbindung gemäß Formel (II) oder dieser selbst durch Tauchen oder Besprühen bei 40 - 90°C weiter imprägniert und das so erhaltene Material gegebenenfalls nach einem Waschvorgang mit einem aliphatischen Alkohol bei Temperaturen zwischen 70 und 250°C trocknet.

**2.** Verfahren gemäß Anspruch 1,
**dadurch gekennzeichnet,**
daß man das Trägermaterial mit einer Lösung imprägniert, die sowohl (eine) Verbindung(en) gemäß Formel (IV) als auch Verbindungen gemäßem Formel (I) enthält.

**3.** Verfahren gemäß Anspruch 1,
**dadurch gekennzeichnet,**
daß im Anschluß an die Stufe a) auch die gemäß Stufe b) einzusetzende Lösung (eine) Verbindung(en) gemäß Formel (IV) enthält.

**4.** Verfahren gemäß Anspruch 1,
**dadurch gekennzeichnet,**

daß man die Stufen b) und c) zusammenfaßt und das Trägermaterial mit einer Lösung der Verbindungen gemäß den Formeln (I) und (II) imprägniert und gemäß Stufe c) weiterbehandelt.

**5.** Verfahren gemäß den Ansprüchen 1 bis 4,
**dadurch gekennzeichnet,**
daß man den Feststoff mit den Verbindungen gemäß den Formeln (I) und (II) in einem molaren Verhältnis von 1 : 10 bis 10 : 1 umsetzt.

## Claims

**1.** Process for the production of oxide and silicate based solids modified on the surface with difunctional organosilicon compounds,
wherein the organosilicon compounds are of the general formula

$$[(RO)_ySi\text{-}R^1\text{-}SO_3^-]_xM^{x+} \qquad (I)$$

and of the general formula

$$(RO)_ySi\text{-}R^2\text{-}SH \qquad (II)$$

in which:

$R^1$ means a linear or branched alkylene group having 1 to 12 C atoms, a cycloalkylene group having 5 to 8 C atoms

or a unit of the general formulae

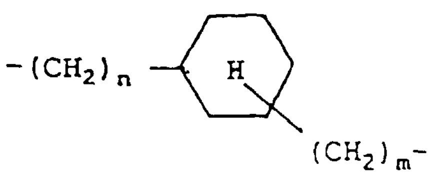
or
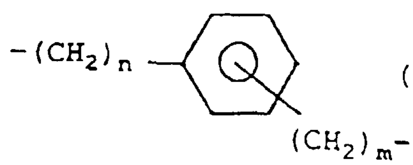
(III)

in which n or m is a number from 0 to 6 and states the number of methylene groups in silicon or sulfur position,
$M^-$ equals $H^-$ or optionally $NH_4^-$ or a metal ion having a valency of x equals 1 to 4, wherein $H^+$ is always present in an amount of at least 5% of the ion exchange capacity of (I),
y is an integer between 1 and 3,

$R^2$ is identical or different from $R^1$ and also has the same meanings as $R^1$ above
R is identical or different and means methyl, ethyl or propyl, H.

characterised in that

a) the solid in the form of mouldings (support material) is optionally impregnated by immersion or spraying with a compound of the general formula

$$Me(OR)_{2\text{-}4}R_{0\text{-}2} \text{ or } Me\,(OR)_{2\text{-}3}R_{0\text{-}1} \qquad (IV),$$

Me: Si, Ti, Al
R: as above

once separated from any possible liquid phase, the solid is dried at 60 to 140°C and

b) the support material is reacted at a temperature of 20 to 140°C with an aqueous solution of organosilicon compounds according to the formula (I) by immersion in the solution or by spraying therewith at standard pressure or a pressure corresponding to the sum of the partial pressures prevailing at the particular temperature, the impregnated support material is separated and optionally dried at 60 to 140°C, optionally hydrothermally post-treated and

c) the resultant support material is further impregnated with a solution of an organosilicon compound according to the formula (II) or with the compound itself by immersion or spraying at 40-90°C and the resultant material, after an optional washing operation with an aliphatic alcohol, is dried at temperatures of between 70 and 250°C.

**2.** Process according to claim 1,
characterised in that
the support material is impregnated with a solution which contains both (a) compound(s) according to the formula (IV) and compounds according to the formula (I).

**3.** Process according to claim 1,
characterised in that after stage a), the solution to be used according to stage b) also contains (a) compound(s) according to the formula (IV).

**4.** Process according to claim 1,
characterised in that
stages b) and c) are combined and the support material is impregnated with a solution of the compounds according to the formulae (I) and (II) and further treated according to stage c).

**5.** Process according to claims 1 to 4,
characterised in that
the solid is reacted with the compounds according to the formulae (I) and (II) in a molar ratio of 1:10 to 10:1.

## Revendications

**1.** Procédé pour la préparation de substances solides d'oxydes et de silicates, modifiées en surface par des composés organosiliciés bifonctionnels, où il s'agit de composés organosiliciés de formule générale

$$[(RO)_ySi\text{-}R^1\text{-}SO^-_3]_xM^{x+} \quad (I)$$

et de formule générale

$$(RO)_ySi\text{-}R^2\text{-}SH \quad (II)$$

dans lesquelles
$R^1$ signifie un groupe alkylène linéaire ou ramifié comprenant 1 à 12 atomes de carbone, un groupe cycloalkylène comprenant 5 à 8 atomes de carbone
ou une unité selon les formules générales

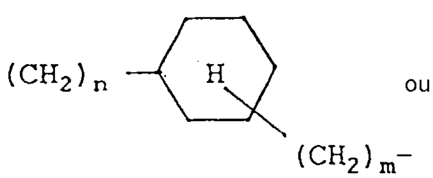

ou

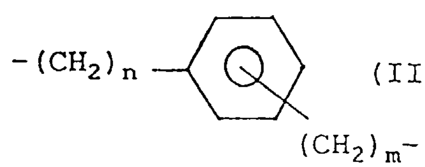

(III)

dans lesquelles

n respectivement m est un nombre de 0 à 6 et indique le nombre de groupes méthylène situés sur le silicium ou le soufre,
$M^+$ est égal à $H^+$ ou, le cas échéant, à $NH_4^+$ ou un ion métallique présentant une valence x entre 1 et 4, $H^+$ étant toujours présent en une quantité représentant au moins 5 % de la capacité d'échange d'ions de (1),
y est un nombre entier entre 1 et 3,
$R^2$ est identique ou différent de $R^1$ et présente les mêmes significations que $R^1$ ci-dessus,
R est identique ou différent et représente le reste méthyle, éthyle ou propyle ou H,

caractérisé

a) en ce qu'on imprègne le cas échéant la substance solide (matériau de support) se trouvant sous forme de corps moulé, par immersion ou pulvérisation avec un composé de formule générale

$$Me(OR)_{2\text{-}4}R_{0\text{-}2} \text{ respectivement } Me(OR)_{2\text{-}3}R_{0\text{-}1} \qquad (IV)$$

avec Me = Si, Ti, Al R tel que défini ci-dessus, en ce qu'on la sèche entre 60 et 140 °C après séparation de la phase liquide éventuellement présente et
b) en ce qu'on transforme le matériau de support à une température de 20 à 140 °C avec une solution aqueuse de composés organosiliciés selon la formule (I) par immersion dans la solution ou pulvérisation avec celle-ci, sous pression normale ou une pression, qui correspond à la somme des pressions partielles qui se règle à la température donnée, en ce qu'on sépare le matériau de support imprégné et en ce qu'on sèche le cas échéant entre 60 et 140 °C, le cas échéant en ce qu'on soumet à un traitement hydrothermique et
c) en ce qu'on imprègne le matériau de support ainsi obtenu avec une solution d'un composé organosilicié selon la formule (II) ou avec le composé lui-même par immersion ou pulvérisation à 40 à 90 °C et en ce qu'on sèche le matériau ainsi obtenu à des températures entre 70 et 250 °C, le cas échéant après une étape de lavage avec un alcool aliphatique.

**2.** Procédé selon la revendication 1, caractérisé en ce qu'on imprègne le matériau de support avec une solution qui contient tant un ou plusieurs composés selon la formule (IV) que des composés selon la formule (I).

**3.** Procédé selon la revendication 1, caractérisé en ce que, consécutivement à l'étape a), la solution à utiliser selon l'étape b) contient également un ou plusieurs composés selon la formule (IV).

**4.** Procédé selon la revendication 1, caractérisé en ce qu'on rassemble les étapes b) et c) et en ce qu'on imprègne le matériau de support avec une solution des composés selon les formules (I) et (II) et en ce qu'on le traite ensuite selon l'étape c).

**5.** Procédé selon les revendications 1 à 4, caractérisé en ce qu'on transforme la substance solide avec les composés selon les formules (I) et (II) dans un rapport molaire de 1:10 à 10:1.